# EUROPÄISCHE PATENTSCHRIFT

(11) **EP 0 956 837 B1**
(45) Veröffentlichungstag und Bekanntmachung des Hinweises auf die Patenterteilung: **13.11.2002**
(21) Anmeldenummer: 99108983.0
(22) Anmeldetag: 06.05.1999
(51) Int. Cl.: A61F 5/01

(54) **Gelenkstütze mit Zahnrad-Verstellmechanismus für die stufenlose Feinverstellung einer Schwenkbereichsgrenze**
Joint brace with gearwheel adjustment mechanism for the continuously variable fine adjustment of a pivot limit
Orthèse d'articulation avec mécanisme de réglage à roue dentée pour règler finement et continûment une limite de pivot

(30) Priorität: 15.05.1998 DE 19821950
(43) Veröffentlichungstag der Anmeldung: 17.11.1999
(73) Patentinhaber: Albrecht GmbH, 83115 Neubeuern (DE)
(72) Erfinder: Albrecht, Erich, 83115 Neubeuern (DE); Opahle, Hans Georg, 83024 Rosenheim (DE)
(74) Vertreter: Bauer, Friedrich

(56) Entgegenhaltungen:
- EP-A- 0 790 046
- US-A- 5 460 599

## Beschreibung

Die Erfindung betrifft eine Gelenkstütze, insbesondere Kniegelenkstütze, gemäß dem Oberbegriff des Anspruches 1.

Gelenkstützen dieser Art sind als Knieorthesen bekannt, die beispielsweise nach Kreuzbandoperationen angelegt werden und eine Bewegung des Unterschenkels relativ zum Oberschenkel unter kontrollierten Bedingungen erlauben sowie das Knie stabilisieren. Die beidseits des Knies entlanggeführten Schienen sind in Höhe des Kniegelenks gelenkig verbunden und werden zu ihren freien Enden hin am Ober- bzw. Unterschenkel festgegurtet. Das Drehgelenk erlaubt hierbei ein Abknicken bzw. Strecken des Beins über einen bestimmten, festlegbaren Schwenkbereich.

Aus der DE 196 06 092 A1 ist eine Gelenkstütze der eingangs genannten Art bekannt, bei welcher ein Verstellhebel, an welchem ein Gegenanschlag zur Begrenzung des freien Schwenkbereiches ausgebildet ist, ein Langloch aufweist, das in einem bestimmten Winkel zu einem Langloch der distalen Schiene verläuft. Durch die beiden Langlöcher ist eine Klemmschraube hindurchgeführt. Durch Verschieben der Klemmschraube innerhalb der Langlöcher kann der Verstellhebel und damit der Gegenanschlag um die Schwenkachse der Gelenkstütze herum sehr fein und stufenlos verschwenkt werden. Hierbei ist es möglich, die Schiene soweit in Extensionsrichtung zu verschwenken, daß ein Überstrecken der Gliedmaßen erreicht wird. Weiterhin ist durch diese Anordnung gewährleistet, daß der Verstellhebel allein durch leichtes Anziehen der Klemmschraube an der distalen Schiene sicher festgelegt werden kann. Diese bekannte Gelenkstütze liefert bereits viele Vorteile gegenüber anderen bekannten Gelenkstützen, erfordert jedoch zum Lösen und Festziehen der Klemmschraube ein Werkzeug in der Form eines Imbusschlüssels. Ein derartiges Werkzeug ist jedoch nicht immer dann zur Hand, wenn die Schwenkbereichsgrenze verstellt werden soll.

Der Erfindung liegt daher die Aufgabe zugrunde, eine Gelenkstütze der eingangs genannten Art zu schaffen, mit der die Verstellung des Verstellhebels relativ zur distalen Schiene auf möglichst einfache Weise und ohne Werkzeug durchgeführt werden kann.

Diese Aufgabe wird erfindungsgemäß durch die Merkmale des Anspruches 1 gelöst. Vorteilhafte Ausführungsformen der Erfindung sind in den weiteren Ansprüchen beschrieben.

Bei der erfindungsgemäßen Gelenkstütze besteht der Verstellmechanismus aus einem manuell betätigbaren Zahnradgetriebe mit einem an der distalen Schiene drehgelagerten Antriebszahnrad, das mit einer am Verstellhebel vorgesehenen Verzahnung in Eingriff ist.

Mit Hilfe des erfindungsgemäßen Verstellmechanismus ist der Verstellhebel und damit der am Verstellhebel vorgesehene Gegenanschlag auf einfache Weise mit den Fingern stufenlos verschwenkbar, ohne daß irgendwelche Werkzeuge benötigt werden. Die Schwenkbereichsgrenze, die beim Anschlagen des Verstellhebel-Gegenanschlags am Anschlagelement der proximalen Schiene erreicht wird, kann somit rein manuell und stufenlos innerhalb eines bestimmten Winkel- bereiches, beispielsweise innerhalb von fünfzehn Grad, verstellt werden.

Zweckmäßigerweise besteht der Verstellmechanismus aus einem selbsthemmenden Zahngetriebe, insbesondere in der Form einer drehbaren Schnecke und eines am Verstellhebel befestigten Schneckenradsegments. Mittels eines derartigen Zahnradgetriebes kann der Verstellhebel sehr fein und leicht verstellt werden, wobei ein derartiges Zahnradgetriebe weiterhin den besonderen Vorteil der Selbsthemmung aufweist. Dies bedeutet, daß durch die Belastung, die durch die Körperglieder auf die Schienen aufgebracht wird, keine Verstellung des Verstellhebels möglich ist. Zusätzliche Mittel zum Sperren des Zahnradgetriebes entfallen damit.

Alternativ hierzu ist es jedoch auch ohne weiteres möglich, anstelle eines Schnecken-Schneckenrad-Getriebes andere manuell betätigbare Zahnradgetriebe zu verwenden und bei fehlender oder ungenügender Selbsthemmung geeignete Sperrglieder vorzusehen, die manuell in und außer Eingriff mit dem Zahnradgetriebe gebracht werden können.

Eine besonders einfache Ausführungsform ergibt sich, wenn das antreibende Getriebeteil eine quer zur Längsachse der distalen Schiene verlaufende, an der distalen Schiene drehbar gelagerte Welle aufweist, welche das Antriebszahnrad, insbesondere die Schnecke, trägt. Eine derartige Welle kann in zumindest einem ihrer Endbereiche, vorzugsweise in beiden Endbereichen, ein mit den Fingern in Umdrehung versetzbares Rändelrad aufweisen, über welches das Antriebszahnrad in Umdrehung versetzt wird.

Die Erfindung wird nachfolgend anhand der Zeichnungen beispielshaft näher erläutert. In diesen zeigen:
- Figur 1 :: eine Draufsicht auf die erfindungsgemäße Gelenkstütze, wobei die Schienen etwas verkürzt dargestellt sind,
- Figur 2 :: einen Schnitt längs der Linie II-II von Figur 1 in vergrößertem Maßstab,
- Figur 3 :: eine Draufsicht auf die erfindungsgemäße Gelenkstütze ohne obere Lochscheibe und ohne obere Lagerschale für den Verstellmechanismus,
- Figur 4 :: einen Schnitt längs der Linie IV-IV von Figur 3 in vergrößertem Maßstab,
- Figur 5 : eine Draufsicht auf eine distale Schiene,
- Figur 6 :: eine Draufsicht auf einen Verstellhebel,
- Figur 7 :: eine Draufsicht auf den erfindungsgemäßen Verstellmechanismus,
- Figur 8 :: einen Schnitt längs der Linie VIII-VIII von Figur 7,
- Figur 9 :: eine Draufsicht auf die untere Lagerschale des Verstellmechanismus,
- Figur 10 :: einen Schnitt längs der Linie X-X von Figur 9,
- Figur 11 :: eine Draufsicht auf die obere Lagerschale des Verstellmechanismus, und
- Figur 12 .: einen Schnitt längs der Linie XII-XII von Figur 11.

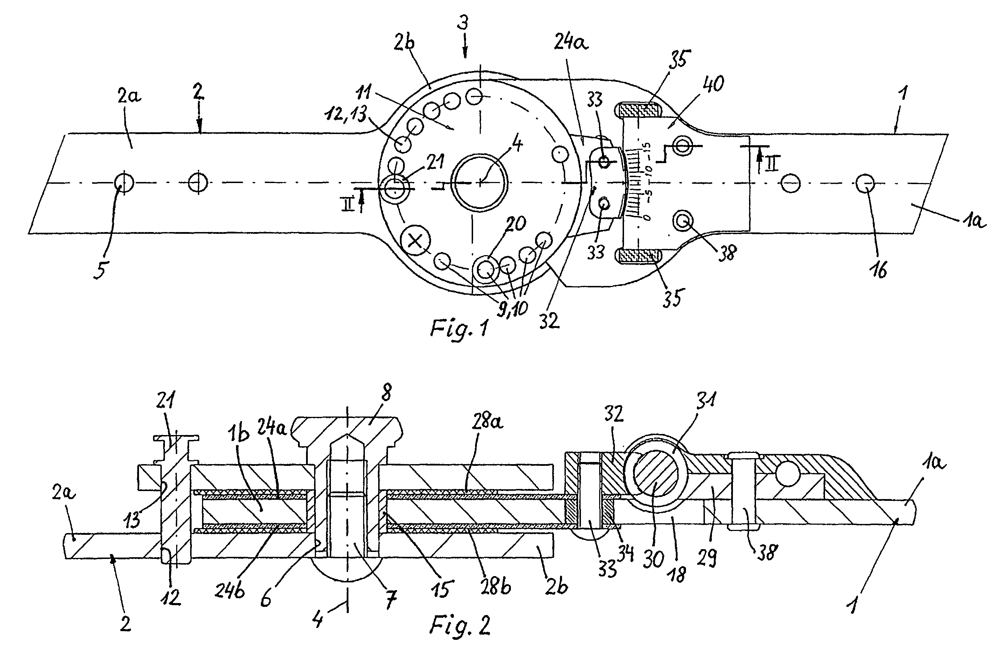

Im folgenden wird anhand der Figuren 1 bis 12 die erfindungsgemäße Gelenkstütze beschrieben, wobei im Normalfall zwei Gelenkstützen der dargestellten Art verwendet werden, die sich auf den gegenüberliegenden Seiten des Gelenks befinden und mittels nicht dargestellter Gurte an den entsprechenden Körpergliedern, beispielsweise dem Oberund Unterschenkel, befestigt werden. Die auf der gegenüberliegenden Seite des Gelenks anzuordnende Gelenkstütze ist spiegelbildlich ausgebildet. Die erfindungsgemäße Gelenkstütze wird anhand des Beispiels einer Knieorthese beschrieben, könnte jedoch auch für andere Gelenke, beispielsweise für das Ellbogengelenk, Anwendung finden.

Wie aus den Figuren 1 bis 4 ersichtlich, besteht die erfindungsgemäße Gelenkstütze aus einer distalen Schiene 1, welche mittels entsprechender, nicht gezeigter Gurte am Unterschenkel befestigt werden kann, sowie einer proximalen Schiene 2, welche ebenfalls mittels nicht gezeigter Gurte am Oberschenkel befestigbar und über ein Gelenk 3 mit der distalen Schiene 1 verbunden ist. Die Schwenkachse ist hierbei mit dem Bezugszeichen 4 bezeichnet.

Die proximale Schiene 2 weist einen länglichen Befestigungsabschnitt 2 und einen kreisscheibenförmigen Gelenkabschnitt 2b auf. Im Befestigungsabschnitt 2b sind mehrere Durchgangslöcher 5 zur Befestigung der nicht dargestellten Gurte vorgesehen.

Der kreisscheibenförmige Gelenkabschnitt 2b weist in seinem Zentrum ein Loch 6 zum Durchtritt einer Schraube 7 und einer Hülsenmutter 8 auf, die in der Schwenkachse 4 liegen. In der Nähe des Außenumfangs sind weiterhin fünf Extensionsbegrenzungslöcher 9 vorgesehen, die fluchtend zu Extensionsbegrenzungslöchern 10 einer oberen Lochscheibe 11 angeordnet sind. Vier Extensionsbegrenzungslöcher 9, 10 sind hierbei in Umfangsrichtung gleichmäßig beabstandet und schließen zueinander einen Winkel von fünfzehn Grad ein.

Auf der anderen Seite des mittigen Lochs 6 sind im Gelenkabschnitt 2b der proximalen Schiene 2 insgesamt sieben Flexionsbegrenzungslöcher 12 vorgesehen, die zu sieben Flexionsbegrenzungslöchern 13 der oberen Lochscheibe 11 fluchten. Zwei nebeneinanderliegende Flexionsbegrenzungslöcher 12, 13 sind wiederum mit einem Winkel von jeweils fünfzehn Grad voneinander beabstandet.

In die Extensionsbegrenzungslöcher 9, 10 und Flexionsbegrenzungslöcher 12, 13 können entsprechende Anschlagelemente 20, 21 eingesteckt werden, um den Schwenkbereich der distalen Schiene 1 gegenüber der proximalen Schiene 2 in Extensions- bzw. Flexionsrichtung zu begrenzen, wie später noch näher erläutert wird.

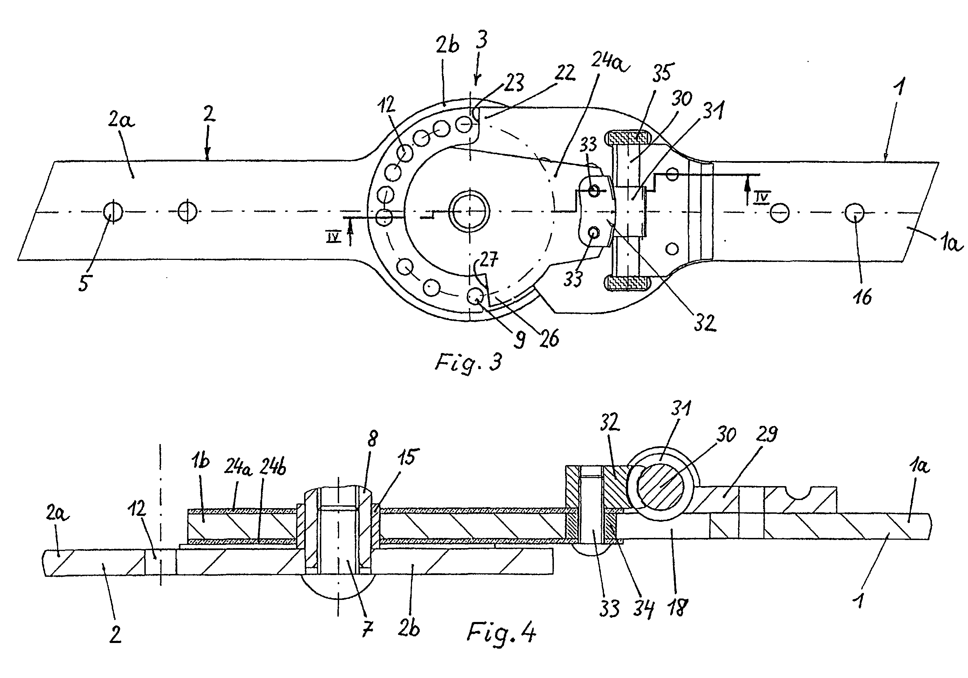

Zwischen dem Gelenkabschnitt 2b der proximalen Schiene 2 und der hierzu parallel mit Abstand angeordneten kreisförmigen Lochscheibe 11 erstreckt sich ein Gelenkabschnitt 1b der distalen Schiene 1, wobei dieser Gelenkabschnitt 1b zu etwas mehr als der Hälfte seines Umfangs kreisförmig ausgebildet ist. In der Mitte des Gelenkabschnitts 1b befindet sich wiederum ein Loch 14, durch das die Hülsenmutter 8 bzw. eine auf dieser Hülsenmutter 8 aufgeschobene Distanzhülse 15 hindurchdritt (Figuren 2 und 4).

Die distale Schiene 1 ist in ähnlicher Weise wie die proximale Schiene 2 über einen länglichen Befestigungsabschnitt 1a mittels nicht dargestellter Gurte befestigbar, wobei zum Anbringen dieser Gurte Durchgangslöcher 16 im Befestigungsabschnitt 1a dienen.

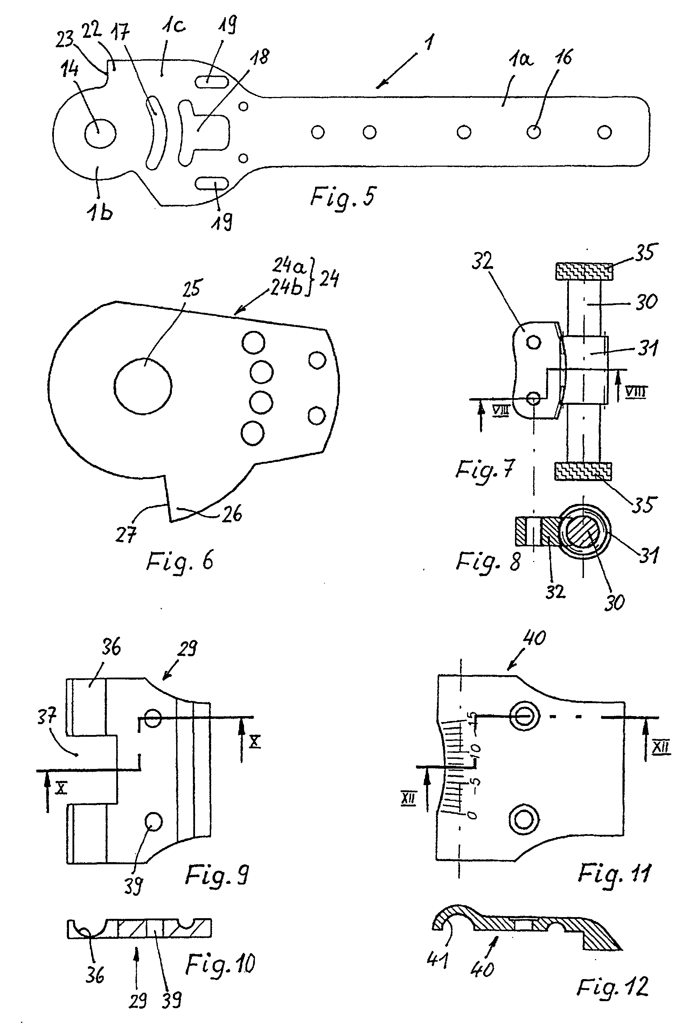

Zwischen dem Befestigungsabschnitt 1a und dem Gelenkabschnitt 1b der distalen Schiene 1 befindet sich ein verbreiterter Übergangsabschnitt 1c, in welchem sich ein kreisbogenförmiges Langloch 17, eine Aussparung 18 sowie zwei Längsaussparungen 19 befinden (Figur 5). Das bogenförmige Langloch 17 und die Aussparung 18 sind hierbei mittig zur zentralen Längsmittellinie der distalen Schiene 1 angeordnet, während sich die Längsaussparungen 19 auf gegenüberliegenden Seiten der Aussparung 18 befinden.

Durch eine nach außen vorspringende Nase 22 der distalen Schiene 1 wird eine Anschlagfläche 23 gebildet, die am Anschlagelement 21 anschlägt, um den Schwenkbereich der distalen Schiene 1 in Flexionsrichtung zu begrenzen.

Wie aus den Figuren 2 und 4 ersichtlich ist, befindet sich auf der Ober- und Unterseite des Gelenkabschnitts 1b der distalen Schiene 1 jeweils ein Verstellhebelblech 24a, 24b, das in Figur 6 einzeln dargestellt ist. Die beiden Verstellhebelbleche 24a, 24b bilden zusammen einen Verstellhebel 24, der relativ zur distalen Schiene 1 um die Schwenkachse 4 herum schwenkbar ist. Hierzu weisen die Verstellhebelbleche 24a, 24b ein Loch 25 auf, durch das die Distanzhülse 15 hindurchtritt. Durch eine seitlich vorspringende Nase wird ein Gegenanschlag 26 mit einer Anschlagfläche 27 gebildet. Die Anschlagfläche 27 schlägt am Anschlagelement 20 an, wenn die distale Schiene 1 maximal in Extensionsrichtung verschwenkt wird, und begrenzt hierdurch den Schwenkbereich in Extensionsrichtung.

Wie aus Figur 2 weiter ersichtlich ist, befindet sich zwischen dem oberen Verstellhebelblech 24a und der Lochscheibe 11 sowie zwischen dem unteren Verstellhebelblech 24b und dem Gelenkabschnitt 2b der proximalen Schiene 2 jeweils eine Reibungsverminderungsscheibe 28a bzw. 28b, beispielsweise in der Form von dünnen Teflonscheiben.

Aufgrund der verschiedenen Anordnungen kann die distale Schiene 1 zusammen mit dem Verstellhebel 24 um die Achse 4 relativ zur proximalen Schiene 2 und der Lochscheibe 11 verschwenkt werden. Die Schwenkbewegung in Flexionsrichtung wird hierbei durch den Ort bestimmt, an welchem die Anschlagfläche 23 der distalen Schiene 1 am Anschlagelement 21 anschlägt, und kann durch Einstecken des Anschlagelements 21 in verschiedene Flexionsbegrenzungslöcher 12, 13 in Stufen von 15° variiert werden. Die Schwenkbewegung in Extensionsrichtung wird durch den Ort begrenzt, an dem die Anschlagfläche 27 der Verstellhebelbleche 24a, 24b am Anschlagelement 20 anschlägt, und kann durch das Einstekken des Anschlagelements 20 in verschiedene Extensionsbegrenzungslöcher 9, 10 in Stufen von 15° variiert werden.

Zusätzlich zu dieser stufenweisen Variierung der Schwenkbereichsgrenze in Extensionsrichtung kann bei dem dargestellten Ausführungsbeispiel eine weitere stufenlose Veränderung der Schwenkbereichsgrenze in Extensionsrichtung durchgeführt werden, indem die Verstellhebelbleche 24a, 24b relativ zur distalen Schiene 1 verschwenkt werden.

Hierzu ist ein Verstellmechanismus nach Art eines Schnecken-Schneckenrad-Antriebs vorgesehen. Dieser Verstellmechanismus umfaßt, wie aus den Figuren 1 bis 4 und 7, 8 ersichtlich ist, eine in einer unteren Lagerschale 29 der distalen Schiene 1 drehbar gelagerte, quer zur Längsrichtung der distalen Schiene 1 angeordnete Welle 30, die mittig eine Schnecke 31 trägt. Diese Schnecke 31 ist mit einem Schneckenradsegment 32 in Eingriff, das am distalen Ende der Verstellhebelbleche 24a, 24b mittels zwei Schrauben 33 befestigt ist. Das Schneckenradsegment 32 befindet sich hierbei radial außerhalb der Lochscheibe 11 und sitzt auf dem oberen Verstellhebelblech 24a auf. Damit die beiden Verstellhebelbleche 24a, 24b durch die Schrauben 33 nicht zusammengedrückt und verformt werden, ist zwischen ihnen eine Distanzhülse 34 vorgesehen. Das Drehen der Schnecke 31 erfolgt manuell mittels zweier Rändelräder 35, die in den beiden Endbereichen der Welle 30 vorgesehen sind. Wird die Schnecke 31 gedreht, bewegt sich das Schneckenradsegment 32 und damit die beiden Verstellhebelbleche 24a, 24b relativ zur distalen Schiene 1 um die Schwenkachse 4.

Die Schraube 33 bzw. die Distanzhülse 34 befinden sich hierbei im Bereich der Aussparung 18 der distalen Schiene 1, so daß sich diese Teile innerhalb der Aussparung 18 bewegen können. Weiterhin greift auch die Schnecke 30 noch teilweise in die Aussparung 18 ein, so daß sich die Schnecke 31 und damit die Welle 30 quer zur Längsrichtung der distalen Schiene 1 nicht verschieben kann. Die Rändelräder 35 greifen in die Längsaussparungen 19 der distalen Schiene 1 ein, wodurch auch eine niedrige Bauhöhe erreicht werden kann.

Die in den Figuren 9 und 10 näher dargestellte untere Lagerschale 29 besteht zweckmäßigerweise aus einem flachen, plattenartigen Kunststoffteil, in dem von oben eine über die gesamte Breite der Lagerschale 29 verlaufende Querrille 36 mit halbkreisförmigem Querschnitt eingebracht ist. In diese Querrille 36 wird die Welle 30 eingesetzt. Durch eine mittige Aussparung 37 wird der für die Schnecke 31 benötigte Freiraum nach unten geschaffen. Die Befestigung der unteren Lagerschale 29 erfolgt bei dem dargestellten Ausführungsbeispiel über Nieten 38 (Figur 2), die durch entsprechende Löcher 39 der unteren Lagerschale 29 hindurchgeführt sind.

Um die Welle 30 auch nach oben festzulegen, ist eine obere Lagerschale 40 vorgesehen, die in den Figuren 11 und 12 in Einzeldarstellung dargestellt ist. Diese obere Lagerschale 40 überdeckt die untere Lagerschale 29 und die Welle 30 mit der Schnecke 31 vollständig und wird ebenfalls mittels der Nieten 38 an der unteren Lagerschale 29 gehalten. Eine der Querrille 36 entsprechende, jedoch entgegengesetzt gekrümmte Querrille 41 mit halbkreisförmigem Querschnittliegt auf der Welle 30 und/oder der Schnecke 31 auf und verhindert deren Abheben nach oben. Weiterhin ist an der Oberseite der oberen Lagerschale 40, wie aus den Figuren 1 und 11 ersichtlich ist, eine Skala vorgesehen, welche einen Winkelbereich von 0 bis 15° umfaßt und ein genaues Ablesen der Relativposition des Schneckenradsegments 32 und damit der Verstellhebelbleche 24a, 24b relativ zur distalen Schiene 1 ermöglicht. Befindet sich das Extensions-Anschlagelement 20 im 0°-Extensionsbegrenzungsloch 9, 10, kann durch die Verstellung der Verstellhebelbleche 24a, 24b die distale Schiene 1 stufenlos bis in einen Überstreckungsbereich von -15° realtiv zur proximalen Schiene 2 abgewinkelt werden.

## Patentansprüche

1. Gelenkstütze, insbesondere Kniegelenkstütze, mit an einem distalen bzw. proximalen Körperglied befestigbaren, mittels eines Gelenks verbundenen Schienen (1), (2), wobei der freie Schwenkbereich der distalen Schiene (1) relativ zur proximalen Schiene (2) mittels eines an der proximalen Schiene (2) in einem schwenkachsennahen Bereich vorgesehenen Anschlagelements (20) begrenzbar ist, der mit einem Gegenanschlag (26) zusammenwirkt, der an einem Verstellhebel (24) vorgesehen ist, der um die Schwenkachse (4) der Gelenkstütze herum schwenkbar gelagert und an der distalen Schiene (1) mittels eines Verstellmechanismus arretierbar sowie in einem bestimmten Winkelbereich stufenlos verstellbar ist, **dadurch gekennzeichnet, daß** der Verstellmechanismus aus einem manuell betätigbaren Zahnradgetriebe (31), (32) mit einem an der distalen Schiene (1) drehgelagerten Antriebszahnrad besteht, das mit einer am Verstellhebel (24) vorgesehenen Verzahnung in Eingriff ist.

2. Gelenkstütze nach Anspruch 1, **dadurch gekennzeichnet, daß** der Verstellmechanismus aus einem selbsthemmenden Zahnradgetriebe besteht.

3. Gelenkstütze nach Anspruch 1 oder 2, **dadurch gekennzeichnet, daß** das antreibende Getriebeteil eine quer zur Längsachse der distalen Schiene (1) verlaufende, an der distalen Schiene (1) drehbar gelagerte Welle (30) aufweist, welche das Antriebszahnrad trägt.

4. Gelenkstütze nach Anspruch 3, **dadurch gekennzeichnet, daß** die Welle (30) in zumindest einem ihrer Endbereiche ein mit den Fingern in Umdrehung versetzbares Rändelrad (35) aufweist.

5. Gelenkstütze nach einem der vorhergehenden Ansprüche, **dadurch gekennzeichnet, daß** die Verzahnung des Verstellhebels (24) an dem zum freien Ende der distalen Schiene (1) hin gerichteten stirnseitigen Ende des Verstellhebels (24) angeordnet ist.

6. Gelenkstütze nach einem der Ansprüche 2 bis 5, **dadurch gekennzeichnet, daß** das Antriebszahnrad aus einer Schnecke (31) und die Verzahnung des Verstellhebels (24) aus einem Schneckenradsegment (32) besteht.

## Claims

1. Joint brace, in particular knee-joint brace, with bars (1), (2) which can be secured on a distal body part and proximal body part, respectively, and are connected by means of a hinge, the free pivoting range of the distal bar (1) relative to the proximal bar (2) being able to be limited by means of a stop element (20) which is provided on the proximal bar (2) in an area near the pivot axis and cooperates with a counterstop (26) which is provided on an adjustment lever (24) which is mounted such that it can be pivoted about the pivot axis (4) of the joint brace and can be locked on the distal bar (1) by means of an adjustment mechanism and can be continuously adjusted within a defined angle range, **characterized in that** the adjustment mechanism consists of a manually actuated toothed gearing (31), (32) with a driving toothed wheel which is mounted rotatably on the distal bar (1) and which is in engagement with a toothing provided on the adjustment lever (24).

2. Joint brace according to Claim 1, **characterized in that** the adjustment mechanism consists of a self-locking toothed gearing.

3. Joint brace according to Claim 1 or 2, **characterized in that** the driving gear part has a shaft (30) which extends transversely with respect to the longitudinal axis of the distal bar (1), is mounted rotatably on the distal bar (1) and carries the driving toothed wheel.

4. Joint brace according to Claim 3, **characterized in that** the shaft (30) has, in at least one of its end areas, a knurled wheel (35) which can be moved in rotation with the fingers.

5. Joint brace according to one of the preceding claims, **characterized in that** the toothing of the adjustment lever (24) is arranged at the front end of the adjustment lever (24) directed towards the free end of the distal bar (1).

6. Joint brace according to one of Claims 2 to 5, **characterized in that** the driving toothed wheel consists of a worm (31) and the toothing of the adjustment lever (24) consists of a worm wheel segment (32).

## Revendications

1. Orthèse d'articulation, en particulier orthèse d'articulation du genou, comportant des tringles (1, 2) susceptibles d'être fixées respectivement sur un membre distal et sur un membre proximal au moyen d'une articulation, la plage de pivotement libre de la tringle distale (1) par rapport à la tringle proximale (2) pouvant être limitée au moyen d'un élément de butée (20) prévu sur la tringle proximale (2) dans une région proche de l'axe de pivotement, lequel élément coopère avec une contre-butée (26) qui est prévue sur un levier de réglage (24) qui est monté pivotant autour de l'axe de pivotement (4) de l'orthèse d'articulation et qui est réglable sur la tringle distale (1) de manière à pouvoir être bloqué au moyen d'un mécanisme de réglage ainsi que de façon continue dans une plage angulaire déterminée, **caractérisée en ce que** le mécanisme de réglage est constitué par une transmission à engrenages (31, 32) à actionnement manuel, comportant un engrenage menant monté rotatif sur la tringle distale (1), l'engrenage étant en engagement avec une denture prévue sur le levier de réglage (24).

2. Orthèse d'articulation selon la revendication 1, **caractérisée en ce que** le mécanisme de réglage est constitué par une transmission à engrenages autobloquante.

3. Orthèse d'articulation selon l'une ou l'autre des revendications 1 et 2, **caractérisée en ce que** la partie de transmission menante présente un arbre (30) monté rotatif sur la tringle distale (1) et s'étendant perpendiculairement à l'axe longitudinal de la tringle distale (1), ledit arbre portant l'engrenage menant.

4. Orthèse d'articulation selon la revendication 3, **caractérisée en ce que** l'arbre (30) présente dans au moins une de ses régions terminales une roue moletée (35) que l'on peut faire tourner avec les doigts.

5. Orthèse d'articulation selon l'une des revendications précédentes, **caractérisée en ce que** la denture du levier de réglage (24) est agencée sur l'extrémité côté frontal du levier de réglage (24) qui est orientée vers l'extrémité libre la tringle distale (1).

6. Orthèse d'articulation selon l'une des revendications 2 à 5, **caractérisée en ce que** l'engrenage menant est constitué par une vis sans fin (31) et **en ce que** la denture du levier de réglage (24) est constituée par un secteur de roue-vis (32).
